# EUROPEAN PATENT SPECIFICATION

(11) **EP 2 124 912 B1**
(45) Date of publication and mention of the grant of the patent: **03.12.2014**
(21) Application number: 07869254.8
(22) Date of filing: 14.12.2007
(51) Int. Cl.: A61K 31/135, A61K 31/55, A61K 31/381, A61K 31/165, A61K 31/496, A61K 31/5375, A61K 31/4178, A61K 31/4164, A61K 45/06

(54) **ALPHA-2B ADRENERGIC RECEPTOR AGONIST AND SEROTONIN-NOREPINEPHRINE REUPTAKE INHIBITOR COMPOSITIONS FOR TREATING CHRONIC PAIN**
ALPHA-2B ADRENERGER REZEPTORAGONIST UND SEROTONIN-NOREPINEPHRIN-WIEDERAUFNAHMEINHIBITOR-ZUSAMMENSETZUNGEN ZUR BEHANDLUNG VON CHRONISCHEN SCHMERZEN
COMPOSITIONS CONTENANT UN AGONISTE DU RÉCEPTEUR ADRÉNERGIQUE ALPHA-2B ET UN INHIBITEUR DU RECAPTAGE DE LA SÉROTONINE-NORÉPINÉPHRINE, DESTINÉES À TRAITER LA DOULEUR CHRONIQUE

(30) Priority: 22.12.2006 US 871715 P
(43) Date of publication of application: 02.12.2009
(60) Divisional application: 11192089.8
(73) Proprietor: ALLERGAN, INC., Irvine, CA 92612 (US)
(72) Inventor: GIL, Daniel W., Corona Del Mar, CA 92625 (US); DONELLO, John E., Dana Point, CA 92629 (US)
(74) Representative: Hoffmann Eitle
(86) International application number: PCT/US2007/087508
(87) International publication number: WO 2008/079728

(56) References cited:
- WO-A-03/053426
- WO-A-03/099289
- WO-A-03/099795
- WO-A-2008/079721
- US-A1- 2006 069 144
- US-B1- 6 329 369
- SCHREIBER S ET AL: "The antinociceptive effect of venlafaxine in mice is mediated through opioid and adrenergic mechanisms" NEUROSCIENCE LETTERS, LIMERICK, IE, vol. 273, 1 January 1999 (1999-01-01), pages 85-88, XP003009174 ISSN: 0304-3940
- BETTUCCI D ET AL: "Combination of tizanidine and amitriptyline in the prophylaxis of chronic tension-type headache: evaluation of efficacy and impact on quality of life" THE JOURNAL OF HEADACHE AND PAIN ; OFFICIAL JOURNAL OF THE ITALIAN SOCIETY FOR THE STUDY OF HEADACHES, SPRINGER-VERLAG, MI, vol. 7, no. 1, 1 February 2006 (2006-02-01), pages 34-36, XP019362030 ISSN: 1129-2377
- MARKOWITZ J S ET AL: "PHARMACOKINETIC AND PHARMACODYNAMIC DRUG INTERACTIONS IN THE TREATMENT OF ATTENTION-DEFICIT HYPERACTIVITY DISORDER" CLINICAL PHARMACOKINETICS, ADIS INTERNATIONAL LTD., AUCKLAND, NZ, vol. 40, no. 10, 1 January 2001 (2001-01-01), pages 753-772, XP008021854 ISSN: 0312-5963

## Description

Disclosed herein is a pharmaceutical composition comprising a serotonin-norepinephrine reuptake inhibitor and an alpha-2B/2C receptor agonist as defined in the claims. The composition is effective for treating chronic pain, and the compounds and composition for use in treating chronic pain are also disclosed. Administering the alpha-2B/2C receptor agonist together with the serotonin- norepinephrine reuptake inhibitor increases the efficacy of the serotonin- norepinephrine reuptake inhibitor in treating pain.

### DETAILED DESCRIPTION OF THE INVENTION

### Serotonin-norepinephrine reuptake inhibitors

The term "serotonin-norepinephrine reuptake inhibitor," as used here, means a composition that inhibits a neuron from reuptaking serotonin and/or norepinephrine after the neuron secretes one of those substances. Hence, the term encompasses three types of compounds: 1) compounds that inhibit neurons from reuptaking serotonin; 2) compounds that inhibit neurons from reuptaking norepinephrine; and 3) compounds that inhibit neurons from reuptaking serotonin and norepinephrine. All three types of compounds may be used in the compositions and methods of the invention.

### Serotonin and Norepinephrine Reuptake Inhibitors

Compounds that inhibit reuptake of both serotonin and norepinephrine, called SNRIs, are well known (S. Schreiber et al, Neuroscience Letters 1999, 273, 85-88, D. Bettucci et al, J. Headache Pain 2006, 7, 34-36, WO 03/053426 A1). They include amitriptyline, desipramine, duloxetine, milnacipran, nefazodone, protriptyline, trimipramine, and venlafaxine. These compounds, including any of their pharmaceutically acceptable salts may be used in the compositions of the invention.

Amitriptyline is an SNRI having the structure

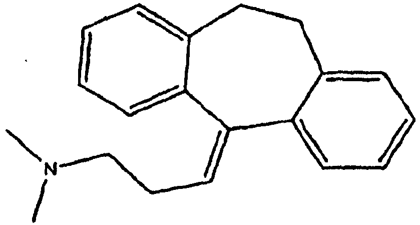

The hydrochloride salt of amitriptyline is sold in the United States under the brand name Elavil®. Amitriptyline is administered to treat depression at an adult oral dose of 50-150 mg every 24 hours, in divided doses, beginning with an initial dose of 25 mg two to four times daily.

Desipramine is an SNRI having the structure

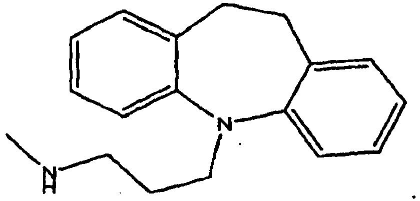

The hydrochloride salt of desipramine is sold in the United States under the brand name Norpramin®. Desipramine is administered to treat depression at an adult dose of 100-200 mg/day, beginning with a lower initial dose. Dosage may be increased to 300 mg/day if necessary.

Duloxetine is an SNRI having the structure

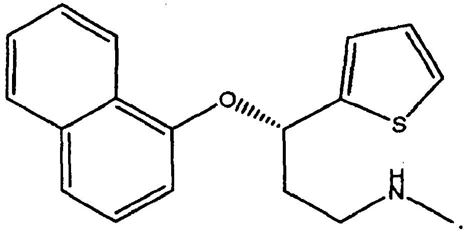

The hydrochloride salt of duloxetine is sold in the United States under the brand name Cymbalta®. Duloxetine is administered to treat depression at an adult dose of 40-60 mg/day, given once daily or as a divided dose twice daily. Doses as high as 120 mg/day are safe but not recommended. Duloxetine is also administered to treat diabetic peripheral neuropathic pain. Its manufacturer (Eli Lilly & Co.) states in the prescribing information that accompanies the product that as "the progression of diabetic peripheral neuropathy is highly variable and management of pain is empirical, the effectiveness of [duloxetine] must be assessed individually." The prescribing information therefore does not suggest a dose for treating diabetic peripheral neuropathy.

Milnacipran is an SNRI having the structure

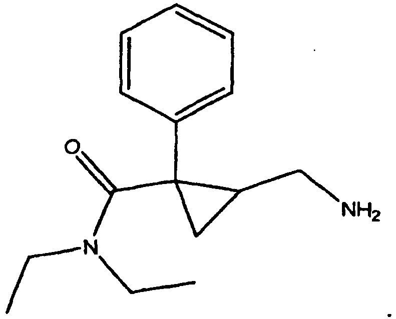

The hydrochloride salt of milnacipran is sold outside the United States under the brand name Ixel. Milnacipran is administered to treat depression at an adult dose of 100 mg, divided over two daily doses; the initial dose is 50 mg once daily. The dosage for the other indications has not been well established so far. Duloxetine is also administered to treat diabetic peripheral neuropathic pain but, as with duloxetine, the dose is patient specific.

Nefazodone is an SNRI having the structure

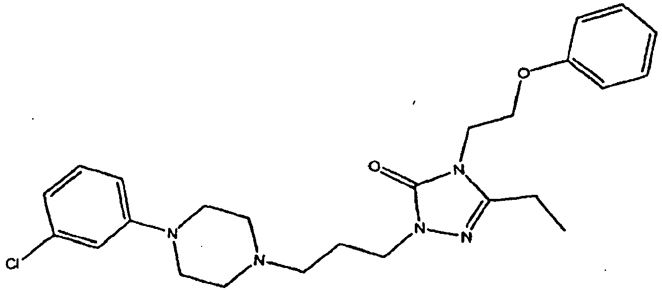

The hydrochloride salt of nefazodone was sold in the United States as Serzone®, but is no longer on the market because of concerns over hepatic failure. Nefazodone was administered to treat depression at an adult dose of 100-600 mg/day, given in divided doses twice daily. The Initial dose was 100 mg and increased, as needed, in 100 mg increments.

Protriptyline is an SNRI having the structure

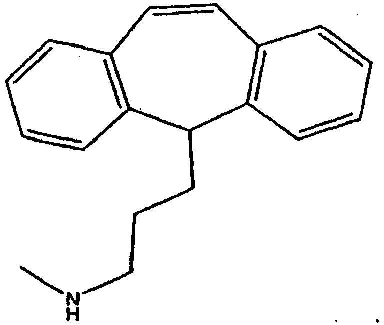

The hydrochloride salt of protriptyline is sold in the United States under the brand name Vivactil®. Protriptyline is administered to treat depression at an adult dose of 15-60 mg/day in divided doses.

Trimipramine is an SNRI having the structure

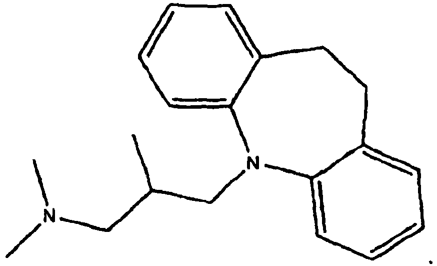

Trimipramine administered to treat depression at an adult dose of 75-200 mg/day, given in two or three divided doses. The Initial dose is 25-100 mg/day and increased by 25 mg/day.

Venlafaxine is an SNRI having the structure

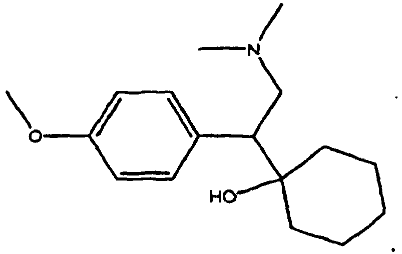

The hydrochloride salt of venlafaxine is sold in the United States under the brand name Effexor®. Venlafaxine is administered to treat depression at an adult dose of 75 mg/day, divided over two or three doses, and is taken with food. If needed, the dose may be increased up to 375 mg/day in increments of 75 mg/day with an interval of at least four days between increments, provided that the drug is well tolerated.

### Norepinephrine Reuptake Inhibitors

Compounds that inhibit norepinephrine reuptake, called norepinephrine reuptake inhibitors, or NRIs, are well known. They include atomoxetine, maprotiline, reboxetine, and viloxazine. These compounds, including any of their pharmaceutically acceptable salts, may be used in the compositions and methods of the invention.

Atomoxetine is an NRI having the structure

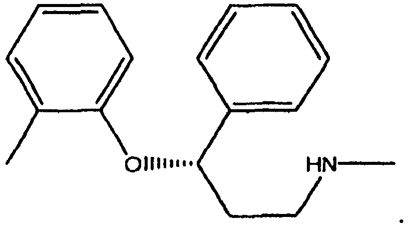

The hydrochloride salt of atomoxetine is sold in the United States under the brand name Strattera®. Atomoxetine is administered to treat attention-deficit/hyperactivity disorder at a pediatric dose of 0.5 - 1.4 mg/kg/day, divided over one or two daily doses, up to a maximum of 100 mg. The initial daily dose is 0.5 mg/kg. The adult dose is 40-100 mg/day, divided over one or two daily doses.

Maprotiline is an NRI having the structure

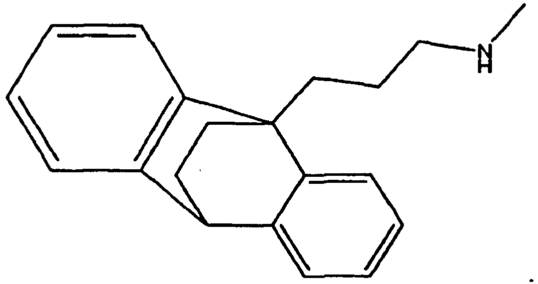

The hydrochloride salt of maprotiline is sold in the United States under the brand name Ludiomil®. Maprotiline is administered to treat depression at an adult dose of 75-225 mg/day, divided over two or three doses. The initial dose is 75 mg.

Reboxetine is an NRI having the structure

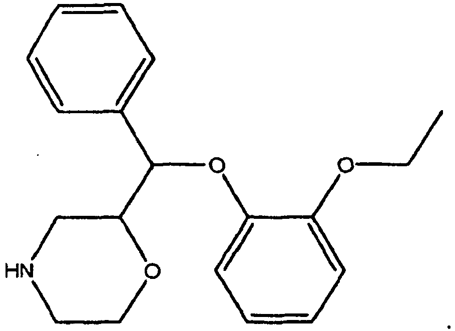

The hydrochloride salt of reboxetine is sold outside the United States under the brand name Edronax. Reboxetine is administered to treat depression at an adult dose of 8-10 mg/day, divided over two doses. The initial dose is 8 mg/day.

Viloxazine is an NRI having the structure

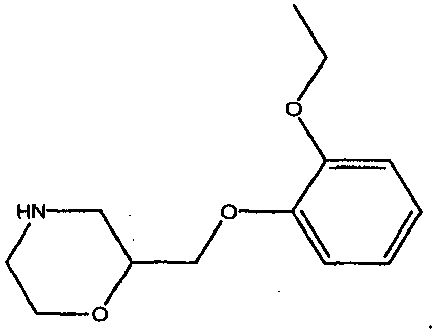

The hydrochloride salt of viloxazine is sold outside the United States under the brand name Vivalan. Viloxazine is administered to treat depression at an adult dose of 150 mg/day.

### Pharmaceutically acceptable salts

Serotonin-norepinephrine reuptake inhibitors may be used as their pharmaceutically acceptable salts.

A "pharmaceutically acceptable salt" is any salt that retains the activity of the parent compound and does not impart any additional deleterious or untoward effects on the subject to which it is administered and in the context in which it is administered compared to the parent compound. A pharmaceutically acceptable salt also refers to any salt which may form in vivo as a result of administration of an acid, another salt, or a prodrug which is converted into an acid or salt.

Pharmaceutically acceptable salts of acidic functional groups may be derived from organic or inorganic bases. The salt may comprise a mono or polyvalent ion. Of particular interest are the inorganic ions lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Hydrochloric acid or some other pharmaceutically acceptable acid may form a salt with a compound that includes a basic group, such as an amine or a pyridine ring.

The serotonin-norepinephrine reuptake inhibitors and alpha-2B/2C receptor agonists of the invention may be either synthetically produced, or may be produced within the body after administration of a prodrug. Hence, "serotonin-norepinephrine reuptake inhibitor" and "alpha-2B/2C receptor agonist" encompass both compounds produced by a manufacturing process and those compounds formed in vivo *only* when another drug administered.

### Isomers and racemates

One can use in the compositions and methods of the invention an enantiomer, sterioisomer, or other isomer of the foregoing compounds (anySNRIor NRI). For example, duloxetine is the S enantiomer of N-methyl-3-(naphthalen-1-yloxy)-3-(thiophen-2-yl)propan-1-amine, but the R enantiomer may also be used; sertraline is the 1S, 4S enantiomer of 4-(3,4-dichlorophenyl)-N-methyl-1,2,3,4-tetrahydronaphthalen-1-amine, but other enantiomers, such as the 1 S, 4R enantiomer or the 1R, 4R enantiomer may be used. One can also use in the compositions and methods of the invention a racemic mixture or one or both racemates, in any proportion. For example, milnacipran is a racemic mixture, but one can use one or both of the racemates, in any proportion.

### Alpha-2B adrenergic receptor agonists

Alpha-2B adrenergic receptor agonists are those compounds that activate to the alpha-2B adrenergic receptor subtype. A compound is an "alpha-2B receptor agonist" if it has greater than 25% efficacy relative to brimonidine at the alpha-2B adrenergic receptor. A compound need not be selective for the alpha-2B adrenergic receptor to be an alpha-2B receptor agonist: the term encompasses agonists that activate alpha-2 adrenergic receptor subtypes other than the alpha-2B receptor subtype and that activate alpha-1 adrenergic receptor subtypes, as well; all such agonists are "alpha-2B receptor agonists" provided that they have greater than 25 % efficacy relative to brimonidine at the alpha-2-B receptor subtype.

In the compositions of the invention alpha-2B receptor agonists that are also alpha-2C receptor agonists are used. A compound is an "alpha-2C receptor agonist" if it has greater than 25% efficacy relative to brimonidine at the alpha-2C receptor. Such an agonist can also be an alpha-2B receptor agonist - an "alpha 2B/2C receptor agonist" - if it also has greater than 25% efficacy relative to brimonidine at the alpha-2B receptor subtype. Note that an agonist can activate the alpha-2C receptor subtype and yet not have 25% efficacy relative to brimonidine at that subtype; such agonists can still be "alpha- 2B receptor agonists," yet are not "alpha-2B/2C receptor agonists" as those terms are defined here.

In the compositions of the invention alpha- 2B/2C receptor agonists lacking significant activity at the alpha-2A receptor subtype are used. An agonist lacks significant alpha-2A receptor activity if the agonist has less than 40% of the efficacy of brimonidine at the alpha-2A receptor subtype.

Efficacy, also known as intrinsic activity, is a measure of maximal receptor activation achieved by a compound and can be determined using any accepted assay of alpha-adrenergic receptor activation, such as a cAMP or Receptor Selection and Amplification Technology (RSAT). Efficacy is represented as a ratio or percentage of the maximal effect of the drug to the maximal effect of a standard agonist for each receptor subtype. Brimonidine, itself an alpha-2B receptor agonist (it is has 100% the efficacy of brimonidine at the alpha-2B adrenergic receptor), is used as the standard agonist for the alpha-2B adrenergic receptors.

Agonist activity can be characterized using any of a variety of routine assays, including, for example, Receptor Selection and Amplification Technology

(RSAT) assays (Messier et al., Pharmacol. Toxicol. 76:308-11 (1995); cyclic AMP assays (Shimizu et al., J. Neurochem. 16:1609-1619 (1969)); and cytosensor microphysiometry assays (Neve et al., J. Biol. Chem. 267:25748-25753 (1992)). Such assays generally are performed using cells that naturally express only a single alpha-adrenergic receptor subtype, or using transfected cells expressing a single recombinant alpha-adrenergic receptor subtype. The adrenergic receptor can be a human receptor or homolog of a human receptor having a similar pharmacology.

The RSAT assay measures receptor-mediated loss of contact inhibition resulting in selective proliferation of receptor-containing cells in a mixed population of confluent cells. The increase in cell number is assessed with an appropriate detectable marker gene such as beta-galactosidase, if desired, in a high throughput or ultra high throughput assay format. Receptors that activate the G protein, Gq, elicit the proliferative response. Alpha-adrenergic receptors, which normally couple to Gi, activate the RSAT response when coexpressed with a hybrid Gq protein containing a Gi receptor recognition domain, designated Gq/i5. Conklin et al., Nature 363:274-6 (1993)).

As an example, an RSAT assay can be performed essentially as follows. NIH-3T3 cells are plated at a density of 2 x 10⁶ cells in 15 cm dishes and maintained in Dulbecco's modified Eagle's medium supplemented with 10% calf serum. One day later, cells are cotransfected by calcium phosphate precipitation with mammalian expression plasmids encoding p-SV-β-galactosidase (5-10 µg), receptor (1-2 µg) and G protein (1-2 µg). Carrier DNA, for example 40 µg salmon sperm DNA, also can be included to increase transfection efficiency. Fresh media is added on the following day; one to two days later, cells are harvested and frozen in 50 assay aliquots. Transfected cells are thawed, and 100 µl of cells added to 100 µl aliquots of compound to be tested, with various concentrations assayed in triplicate, for example, in 96-well plates. Incubation continues for 72 to 96 hours at 37° C. After washing with phosphate-buffered saline, β-galactosidase activity is determined by adding 200 µl of chromogenic substrate (3.5 mM O-nitrophenyl-β-D-galactopyranoside/0.5% NP-40 in phosphate buffered saline), incubating overnight at 30° C, and measuring optical density at 420 nm. The absorbancy is a measure of enzyme activity, which depends on cell number and reflects receptor-mediated cell proliferation. The EC₅₀ and maximal effect (i.e., efficacy) of each drug at each receptor is determined.

Alpha-2B/2C receptor agonists lacking significant activity at the alpha-2A receptor subtye includes the compounds below in Table 1 :

**Table 1 - Alpha-2B/2C receptor agonists lacking significant activity at the alpha-2A receptor subtype**

| **COMPOUND** | **STRUCTURE** |
|---|---|
| 18 | 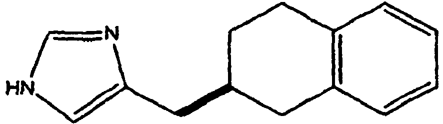 |
| 19 | 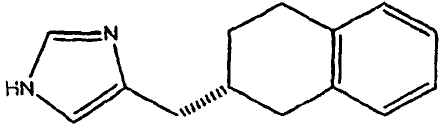 |
| 84 | 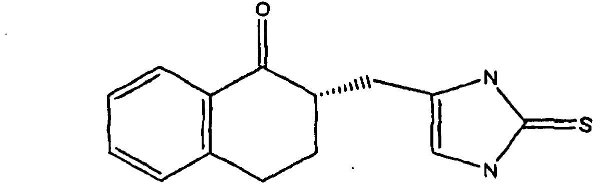 |
| 85 | 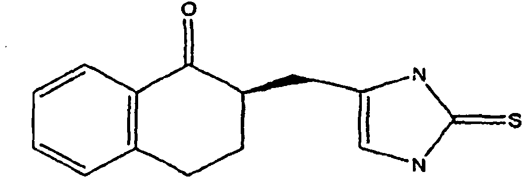 |
| 89 | 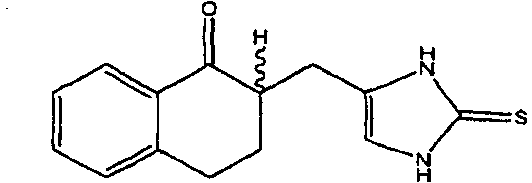 |
| | (-) optically pure |
| 90 | 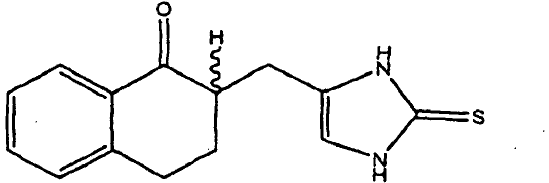 |
| | (+) optically pure |
| 91 | 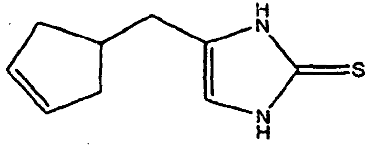 |
| 95 | 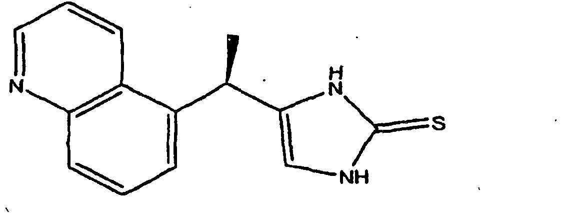 |
| 96 | 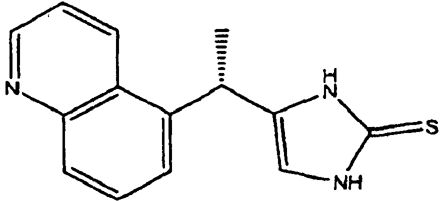 |
| 97 | 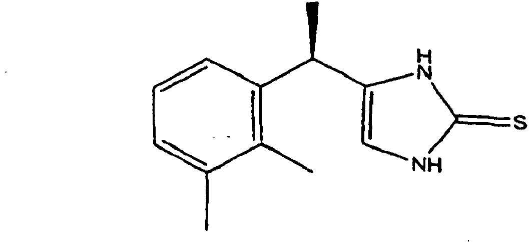 |
| 98 | 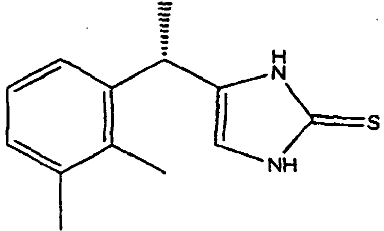 |

Alpha-28 and -2C receptor agonists lacking significant alpha-2A receptor activity are known in the art. Detailed information regarding alpha-2 agonists, including their structure, synthesis, and activity, may be found in U.S. Patent No. 6,329,369, No. 6,534,542, No. 6,545,182, No. 6,787,517, No. 6,841 ,684, and No. 7,091 ,232; in U.S. Patent Application Publication No. 2003/0092766, No. 2004/0132824, No. 2004/0220402, No. 2005/0075366, and No. 2005/0267186; and in U.S. Patent Application No. 11/172,229, No. 11/232,323, No. 11/232,341 , No. 60/613,870, No. 60/695,650, No. 60/747,444, No. 60/884,718, No. 60/917,828, No. 60/911,422, No. 60/911,478, No. 60/948,389, US 2006/0069144 A1, WO 03/099289 A2 and WO 03/099795 A1.

One can use in the compositions of the invention any pharmaceutically acceptable salt, isomer, and racemate (as those terms are defined in the preceding sections) of the alpha-2B/2C receptor agonist lacking significant activity at the alpha-2A receptor subtype specified above.

### Pharmaceutical compositions

Pharmaceutical compositions of the invention comprise one or more serotonin-norepinephrine reuptake inhibitors as specified above and one or more alpha-2B/2C receptor agonists, lacking significant activity at the alpha-2A receptor subtype as specified above.

### Dose

The pharmaceutical compositions of the invention may be formulated such that a patient receives a dose of a serotonin-norepinephrine reuptake inhibitor that is usually effective, when administered separately, to relieve pain, and a dose of an alpha-2B receptor agonist that is usually effective, when administered separately, to relieve pain. But the pharmaceutical compositions of the invention may also be formulated such that doses of each compound may be those that are ineffective or minimally effective when the compounds are administered alone. This allows one to administer to a patient a formulation of the invention that is as effective as a larger dose of a serotonin-norepinephrine reuptake inhibitor administered alone, but less likely to lead to side effects. This does not mean, however, that formulations of the invention comprise serotonin- norepinephrine reuptake inhibitors and alpha-2B receptor agonists in only such doses which are, when administered alone, minimally effective: a patient with severe pain may require a high dose of either component of the formulation, but is still likely to experience enhanced pain relief (as compared to the relief the patient would experience were he administered a high dose of either component of the invention alone).

The precise dose and frequency of administration depends on the severity and nature of the patient's condition, on the manner of administration, on the potency and pharmacodynamics of the particular compound employed, and on the judgment of the prescribing physician. Determining dose is a routine matter that is well within the capability of someone of ordinary skill in the art. The dose of serotonin-norepinephrine reuptake inhibitors effective to treat depression, discussed in previous sections, may moreover be used as a guide.

It may be desirable to administer a dose of each compound that is ineffective or minimally effective when the serotonin-norepinephrine reuptake inhibitors or alpha-2B receptor agonists are administered alone. Determining such a dose is a routine matter. Typical such doses are set forth below:

**Table 2 - Doses of serotonin-norepinephrine reuptake inhibitors that are generally ineffective or minimally effective, when administered alone, to relieve pain in adult patients**

| **COMPOSITION** | **INEFFECTIVE OR MINIMALLY EFFECTIVE DOSES** |
|---|---|
| Amitriptyline | < 50-150 mg/day |
| Desipramine | < 100-200 mg/day |
| Duloxetine | < 40-60 mg/day |
| Milnacipran | < 100 mg/day |
| Nefazodone | < 100-600 mg/day |
| Protriptyline | < 15-60 mg/day |
| Trimipramine | < 75-200 mg/day |
| Venlafaxine | < 75 mg/day |
| Atomoxetine | < 40-100 mg/day |
| Maprotiline | < 75-225 mg/day |
| Reboxetine | < 8-10 mg/day |
| Viloxazine | < 150 mg/day |

### Excipients and dosage forms

Those skilled in the art will readily understand that for administering pharmaceutical compositions of the invention serotonin-norepinephrine reuptake inhibitors and alpha-2B/2C receptor agonists lacking specific activity at the alpha-2A receptor subtype as specified above can be admixed with pharmaceutically acceptable excipient which are well known in the art.

A pharmaceutical composition to be administered systemically may be confected as a powder, pill, tablet or the like, or as a solution, emulsion, suspension, aerosol, syrup or elixir suitable for oral or parenteral administration or inhalation.

For solid dosage forms or medicaments, non-toxic solid carriers include, but are not limited to, pharmaceutical grades of mannitol, lactose, starch, magnesium stearate, sodium saccharin, the polyalkylene glycols, talcum, cellulose, glucose, sucrose and magnesium carbonate. The solid dosage forms may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the technique described in U.S. Patent No. 4,256,108, No. 4,166,452, and No. 4,265,874 to form osmotic therapeutic tablets for control release. Liquid pharmaceutically administrable dosage forms can, for example, comprise a solution or suspension of one or more of the presently useful compounds and optional pharmaceutical adjutants in a carrier, such as for example, water, saline, aqueous dextrose, glycerol, ethanol and the like, to thereby form a solution or suspension. If desired, the pharmaceutical composition to be administered may also contain minor amounts of nontoxic auxiliary substances such as wetting or emulsifying agents, pH buffering agents and the like. Typical examples of such auxiliary agents are sodium acetate, sorbitan monolaurate, triethanolamine, sodium acetate, triethanolamine oleate, etc. Actual methods of preparing such dosage forms are known, or will be apparent, to those skilled in this art; for example, see Remington's Pharmaceutical Sciences, Mack Publishing Company, Easton, Pa., 16th Edition, 1980. The composition of the formulation to be administered, in any event, contains a quantity of one or more of the presently useful compounds in an amount effective to provide the desired therapeutic effect.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol and the like. In addition, if desired, the injectable pharmaceutical compositions to be administered may also contain minor amounts of non-toxic auxiliary substances such as wetting or emulsifying agents, pH buffering agents and the like.

### Compounds and compositions for use in Methods of treatment

The pharmaceutical compositions of the invention may be used to treat chronic pain. To "treat," as used here, means to deal with medically. It includes both preventing pain and relieving it.

Pain, in general, may be divided into two types: chronic and acute. Acute pain has a relatively short duration and sudden onset. One type of acute pain, for example, is cutaneous pain felt on injury to the skin or other superficial tissues, such as caused by a cut or a burn. Cutaneous nociceptors terminate just below the skin, and due to the high concentration of nerve endings, produce a well-defined, localized pain of short duration.

Chronic pain is a pain other than an acute pain. There are various types of chronic pain, but those types of pain most amenable to treatment with the compositions and methods of the invention include neuropathic pain, inflammatory pain, somatic pain, visceral pain, and referred pain.

### I. Neuropathic Pain

The compositions of the invention may be used to treat pain caused by or otherwise associated with any of the following neuropathic pain conditions. "Neuropathic pain" means abnormal sensory input, resulting from injury or malfunction of the peripheral nervous system, central nervous system, or both, that produces pain.

### A. Symptoms of neuropathic pain

Symptoms of neuropathic pain can involve persistent, spontaneous pain, as well as allodynia, hyperalgesia, or hyperpathia.

### B. Causes of neuropathic pain

Neuropathic pain may be caused by any of the following.
1. A traumatic insult, such as, for example, a nerve compression injury (e.g., a nerve crush, a nerve stretch, a nerve entrapment or an incomplete nerve transsection); a spinal cord injury (e.g., a hemisection of the spinal cord); a limb amputation; a contusion; an inflammation (e.g., an inflammation of the spinal cord); or a surgical procedure.
2. An ischemic event, including, for example, a stroke and heart attack.
3. An infectious agent
4. Exposure to a toxin, including, for example, a drug, an alcohol, a heavy metal (e.g., lead, arsenic, mercury), an industrial agent (e.g., a solvent, fumes from a glue) or nitrous oxide.
5. A disease, including, for example, an inflammatory disorder, a neoplastic tumor, an acquired immune deficiency syndrome (AIDS), Lymes disease, a leprosy, a metabolic disease, a neurodegenerative disease, a spinal stenosis, a mononeuropathy, a polyneuropathy, and a peripheral nerve disorder, such as a neuroma.

### C. Types of neuropathic pain

### 1. Neuralgia

A neuralgia is a pain that radiates along the course of one or more specific nerves usually without any demonstrable pathological change in the nerve structure. The causes of neuralgia are varied. Chemical irritation, inflammation, trauma (including surgery), compression by nearby structures (for instance, tumors), and infections may all lead to neuralgia. In many cases, however, the cause is unknown or unidentifiable. Neuralgia is most common in elderly persons, but it may occur at any age. A neuralgia, includes, without limitation, a trigeminal neuralgia, a spinal stenosis, a post-herpetic neuralgia, a postherpetic neuralgia, a glossopharyngeal neuralgia, pain associated with nerve entrapment disorders, a sciatica and an atypical facial pain.

Neuralgia is a painful disorder of the cranial nerves. Falling under the category of neuralgia are trigeminal neuralgia (TN), atypical facial pain, and postherpetic neuralgia (caused by shingles or herpes). The affected nerves are responsible for sensing touch, temperature and pressure in the facial area from the jaw to the forehead. The disorder generally causes short episodes of excruciating pain, usually for less than two minutes and on only one side of the face. The pain can be described in a variety of ways such as "stabbing," "sharp," "like lightning," "burning," and even "itchy". In the atypical form of TN, the pain can also present as severe or merely aching and last for extended periods. The pain associated with TN is recognized as one the most excruciating pains that can be experienced.

Simple stimuli such as eating, talking, washing the face, or any light touch or sensation can trigger an attack (even the sensation of a gentle breeze). The attacks can occur in clusters or as an isolated attack.

Symptoms include sharp, stabbing pain or constant, burning pain located anywhere, usually on or near the surface of the body, in the same location for each episode; pain along the path of a specific nerve; impaired function of affected body part due to pain, or muscle weakness due to concomitant motor nerve damage; increased sensitivity of the skin or numbness of the affected skin area (feeling similar to a local anesthetic such as a Novacaine shot); and any touch or pressure is interpreted as pain. Movement may also be painful.

Trigeminal neuralgia is the most common form of neuralgia. It affects the main sensory nerve of the face, the trigeminal nerve ("trigeminal" literally means "three origins", referring to the division of the nerve into 3 branches). This condition involves sudden and short attacks of severe pain on the side of the face, along the area supplied by the trigeminal nerve on that side. The pain attacks may be severe enough to cause a facial grimace, which is classically referred to as a painful tic (tic douloureux). Sometimes, the cause of trigeminal neuralgia is a blood vessel or small tumor pressing on the nerve. Disorders such as multiple sclerosis (an inflammatory disease affecting the brain and spinal cord), certain forms of arthritis, and diabetes (high blood sugar) may also cause trigeminal neuralgia, but a cause is not always identified. In this condition, certain movements such as chewing, talking, swallowing, or touching an area of the face may trigger a spasm of excruciating pain.

A related but rather uncommon neuralgia affects the glosso-pharyngeal nerve, which provides sensation to the throat. Symptoms of this neuralgia are short, shock-like episodes of pain located in the throat.

Neuralgia may occur after infections such as shingles, which is caused by the varicella-zoster virus, a type of herpesvirus. This neuralgia produces a constant burning pain after the shingles rash has healed. The pain is worsened by movement of or contact with the affected area. Not all of those diagnosed with shingles go on to experience postherpetic neuralgia, which can be more painful than shingles. The pain and sensitivity can last for months or even years. The pain is usually in the form of an intolerable sensitivity to any touch but especially light touch. Postherpetic neuralgia is not restricted to the face; it can occur anywhere on the body but usually occurs at the location of the shingles rash. Depression is not uncommon due to the pain and social isolation during the illness.

Postherpetic neuralgia may be debilitating long after signs of the original herpes infection have disappeared. Other infectious diseases that may cause neuralgia are syphilis and Lyme disease.

Diabetes is another common cause of neuralgia. This very common medical problem affects almost 1 out of every 20 Americans during adulthood. Diabetes damages the tiny arteries that supply circulation to the nerves, resulting in nerve fiber malfunction and sometimes nerve loss. Diabetes can produce almost any neuralgia, including trigeminal neuralgia, carpal tunnel syndrome (pain and numbness of the hand and wrist), and meralgia paresthetica (numbness and pain in the thigh due to damage to the lateral femoral cutaneous nerve). Strict control of blood sugar may prevent diabetic nerve damage and may accelerate recovery in patients who do develop neuralgia.

Other medical conditions that may be associated with neuralgias are chronic renal insufficiency and porphyria - a hereditary disease in which the body cannot rid itself of certain substances produced after the normal breakdown of blood in the body. Certain drugs may also cause this problem.

### 2. Deafferentation.

Deafferentation indicates a loss of the sensory input from a portion of the body, and can be caused by interruption of either peripheral sensory fibres or nerves from the central nervous system. A deafferentation pain syndrome, includes, without limitation, an injury to the brain or spinal cord, a post-stroke pain, a phantom pain, a paraplegia, a brachial plexus avulsion injuries, lumbar radiculopathies.

### 3. Complex regional pain syndromes (CRPSs)

CRPS is a chronic pain syndrome with two forms. CRPS 1 currently replaces the term "reflex sympathetic dystrophy syndrome". It is a chronic nerve disorder that occurs most often in the arms or legs after a minor or major injury. CRPS 1 is associated with severe pain; changes in the nails, bone, and skin; and an increased sensitivity to touch in the affected limb. CRPS 2 replaces the term causalgia, and results from an identified injury to the nerve. A CRPS, includes, without limitation, a CRPS Type I (reflex sympathetic dystrophy) and a CRPS Type II (causalgia).

### 4. Neuropathy.

A neuropathy is a functional or pathological change in a nerve and is characterized clinically by sensory or motor neuron abnormalities.

Central neuropathy is a functional or pathological change in the central nervous system.

Peripheral neuropathy is a functional or pathological change in one or more peripheral nerves. The peripheral nerves relay information from your central nervous system (brain and spinal cord) to muscles and other organs and from your skin, joints, and other organs back to your brain. Peripheral neuropathy occurs when these nerves fail to carry information to and from the brain and spinal cord, resulting in pain, loss of sensation, or inability to control muscles. In some cases, the failure of nerves that control blood vessels, intestines, and other organs results in abnormal blood pressure, digestion problems, and loss of other basic body processes. Risk factors for neuropathy include diabetes, heavy alcohol use, and exposure to certain chemicals and drugs. Some people have a hereditary predisposition for neuropathy. Prolonged pressure on a nerve is another risk for developing a nerve injury. Pressure injury may be caused by prolonged immobility (such as a long surgical procedure or lengthy illness) or compression of a nerve by casts, splints, braces, crutches, or other devices. Polyneuropathy implies a widespread process that usually affects both sides of the body equally. The symptoms depend on which type of nerve is affected. The three main types of nerves are sensory, motor, and autonomic. Neuropathy can affect any one or a combination of all three types of nerves. Symptoms also depend on whether the condition affects the whole body or just one nerve (as from an injury). The cause of chronic inflammatory polyneuropathy is an abnormal immune response. The specific antigens, immune processes, and triggering factors are variable and in many cases are unknown. It may occur in association with other conditions such as HIV, inflammatory bowel disease, lupus erythematosis, chronic active hepatitis, and blood cell abnormalities.

Peripheral neuropathy may involve a function or pathological change to a single nerve or nerve group (monneuropathy) or a function or pathological change affecting multiple nerves (polyneuropathy). Table 1, below, lists some causes of peripheral neuropathies:

**Table 1. Some Causes of Peripheral neuropathies**

| |
|---|
| Hereditary disorders |
| Charcot-Marie-Tooth disease |
| Friedreich's ataxia |
| Systemic or metabolic disorders |
| Diabetes (diabetic neuropathy) |
| Dietary deficiencies (especially vitamin B-12) |
| Excessive alcohol use (alcoholic neuropathy) |
| Uremia (from kidney failure) |
| Cancer (including bone cancer and other cancers) |
| Infectious or inflammatory conditions |
| AIDS |
| Hepatitis |
| Colorado tick fever |
| Diphtheria |
| Guillain-Barre syndrome |
| HIV infection without development of AIDS |
| Leprosy |
| Lyme disease |
| Polyarteritis nodosa |
| Rheumatoid arthritis |
| Sarcoidosis |
| Sjogren's syndrome |
| Syphilis |
| Systemic Lupus erythematosus |
| amyloid |
| Exposure to toxic compounds |
| Sniffing glue or other toxic compounds |
| Nitrous oxide |
| Industrial agents -- especially solvents |
| Heavy metals (lead, arsenic, mercury, etc.) |
| Neuropathy secondary to drugs like analgesic nephropathy |
| Rhabdomyolysis |
| Macrohagic myofasciitis |
| Highly Active Anti-Retrviral Therapy (HAART)-induced neuropathy |
| Chemotherapy Incuced Neuropathy |
| Miscellaneous causes |
| Ischemia (decreased oxygen/decreased blood flow) |
| Prolonged exposure to cold temperature |

### a. Polyneuropathy

Polyneuropathy is a peripheral neuropathy involving the loss of movement or sensation to an area caused by damage or destruction to multiple peripheral nerves. Polyneuropathic pain, includes, without limitation, post-polio syndrome, postmastectomy syndrome, diabetic neuropathy, alcohol neuropathy, amyloidosis, toxin exposure, AIDS, hypothyroidism, uremia, vitamin deficiencies, chemotherapy-induced pain, 2',3'-didexoycytidine (ddC) treatment, exposure to the anticonvulsant phenytoin, exposure to antibiotics including chloramphenicol, nitrofurantoin and sulfonamineds, exposure to sedatives including barbital and hexobarbital, Guillain-Barré syndromes, Fabry's disease or polyneuropathy secondary to cancers such as multiple myeloma.

### b. Mononeuropathy

Mononeuropathy is a peripheral neuropathy involving loss of movement or sensation to an area caused by damage or destruction to a single peripheral nerve or nerve group. Mononeuropathy is most often caused by damage to a local area resulting from injury or trauma, although occasionally systemic disorders may cause isolated nerve damage (as with mononeuritis multiplex). The usual causes are direct trauma, prolonged pressure on the nerve, and compression of the nerve by swelling or injury to nearby body structures. The damage includes destruction of the myelin sheath (covering) of the nerve or of part of the nerve cell (the axon). This damage slows or prevents conduction of impulses through the nerve. Mononeuropathy may involve any part of the body. Mononeuropathic pain, includes, without limitation, a sciatic nerve dysfunction, a common peroneal nerve dysfunction. a radial nerve dysfunction, an ulnar nerve dysfunction, a cranial mononeuropathy VI, a cranial mononeuropathy VII, a cranial mononeuropathy III (compression type), a cranial mononeuropathy III (diabetic type), an axillary nerve dysfunction, a carpal tunnel syndrome, a femoral nerve dysfunction, a tibial nerve dysfunction, a Bell's palsy, a thoracic outlet syndrome, a carpal tunnel syndrome, and a sixth (abducent) nerve palsy.

### c. Generalized peripheral neuropathies

Generalized peripheral neuropathis are symmetrical, and usually due to various systematic illnesses and disease processes that affect the peripheral nervous system in its entirety. They are further subdivided into several categories:
i. Distal axonopathies are the result of some metabolic or toxic derangement of neurons. They may be caused by metabolic diseases such as diabetes, renal failure, deficiency syndromes such as malnutrition and alcoholism, or the effects of toxins or drugs. Distal axonopathy (aka dying back neuropathy) is a type of peripheral neuropathy that results from some metabolic or toxic derangement of peripheral nervous system (PNS) neurons. It is the most common response of nerves to metabolic or toxic disturbances, and as such may be caused by metabolic diseases such as diabetes, renal failure, deficiency syndromes such as malnutrition and alcoholism, or the effects of toxins or drugs. The most common cause of distal axonopathy is diabetes, and the most common distal axonopathy is diabetic neuropathy.
ii. Myelinopathies are due to a primary attack on myelin causing an acute failure of impulse conduction. The most common cause is acute inflammatory demyelinating polyneuropathy (AIDP; aka Guillain-Barré syndrome), though other causes include chronic inflammatory demyelinating syndrome (CIDP), genetic metabolic disorders (e.g., leukodystrophy), or toxins. Myelinopathy is due to primary destruction of myelin or the myelinating Schwann cells, which leaves the axon intact, but causes an acute failure of impulse conduction. This demyelination slows down or completely blocks the conduction of electical impulses through the nerve. The most common cause is acute inflammatory demyelinating polyneuropathy (AIDP, better known as Guillain-Barré syndrome), though other causes include chronic inflammatory demyelinating polyneuropathy (CIDP), genetic metabolic disorders (e.g., leukodystrophy or Charcot-Marie-Tooth disease), or toxins.
iii. Neuronopathies are the result of destruction of peripheral nervous system (PNS) neurons. They may be caused by motor neurone diseases, sensory neuronopathies (e.g., Herpes zoster), toxins or autonomic dysfunction. Neurotoxins may cause neuronopathies, such as the chemotherapy agent vincristine. Neuronopathy is dysfunction due to damage to neurons of the peripheral nervous system (PNS), resulting in a peripheral neuropathy. It may be caused by motor neurone diseases, sensory neuronopathies (*e.g.*, Herpes zoster), toxic substances or autonomic dysfunction. A person with neuronopathy may present in different ways, depending on the cause, the way it affects the nerve cells, and the type of nerve cell that is most affected.
iv. Focal entrapment neuropathies (e.g., carpal tunnel syndrome) represent an additional category of generalized peripheral neuropathies.

### II. Inflammatory pain

The compositions of the invention may be used to treat pain caused by or otherwise associated with any of the following inflammatory conditions.

### A. Arthritic disorder

Arthritic disorders include, for example, a rheumatoid arthritis; a juvenile rheumatoid arthritis; a systemic lupus erythematosus (SLE); a gouty arthritis; a scleroderma; an osteoarthritis; a psoriatic arthritis; an ankylosing spondylitis; a Reiter's syndrome (reactive arthritis); an adult Still's disease; an arthritis from a viral infection; an arthritis from a bacterial infection, such as, e.g., a gonococcal arthritis and a non-gonococcal bacterial arthritis (septic arthritis); a Tertiary Lyme disease; a tuberculous arthritis; and an arthritis from a fungal infection, such as, e.g., a blastomycosis

### B. Autoimmune diseases

Autoimmune diseases include, for example, a Guillain-Barré syndrome, a Hashimoto's thyroiditis, a pernicious anemia, an Addison's disease, a type I diabetes, a systemic lupus erythematosus, a dermatomyositis, Sjogren's syndrome, a lupus erythematosus, a multiple sclerosis, a myasthenia gravis, a Reiter's syndrome, a Grave's disease, and a rheumatoid arthritis.

### C. Connective tissue disorder

Connective tissue disorders include, for example, a spondyloarthritis a dermatomyositis, and a fibromyalgia syndrome.

### D. Injury

Inflammation caused by injury, including, for example, a crush, puncture, stretch of a tissue or joint, may cause chronic inflammatory pain.

### E. Infection

Inflammation caused by infection, including, for example, a tuberculosis or an interstitial keratitis may cause chronic inflammatory pain. Infection may also result in inflammatory bowel diseases and irritable bowel syndromes.

### F. Neuritis

Neuritis is an inflammatory process affecting a nerve or group of nerves. Symptoms depend on the nerves involved, but may include pain, paresthesias, paresis, or hypesthesia (numbness).

Examples include:
a. Brachial neuritis
b. Retrobulbar neuropathy, an inflammatory process affecting the part of the optic nerve lying immediately behind the eyeball.
c. Optic neuropathy, an inflammatory process affecting the optic nerve causing sudden, reduced vision in the affected eye. The cause of optic neuritis is unknown. The sudden inflammation of the optic nerve (the nerve connecting the eye and the brain) leads to swelling and destruction of the myelin sheath. The inflammation may occasionally be the result of a viral infection, or it may be caused by autoimmune diseases such as multiple sclerosis. Risk factors are related to the possible causes.
d. Vestibular neuritis, a viral infection causing an inflammatory process affecting the vestibular nerve.

### G. Joint inflammation

Inflammation of the joint, such as that caused by bursitis or tendonitis, for example, may cause chronic inflammatory pain.

### III. Headache Pain

The compositions of the invention may be used to treat pain caused by or otherwise associated with chronic headache conditions. A headache (medically known as cephalgia) is a condition of mild to severe pain in the head; sometimes neck or upper back pain may also be interpreted as a headache. It may indicate an underlying local or systemic disease or be a disorder in itself.

### IV. Somatic pain

The compositions of the invention may be used to treat pain caused by or otherwise associated with any of the following somatic pain conditions. Somatic pain originates from ligaments, tendons, bones, blood vessels, and even nerves themselves. It is detected with somatic nociceptors. The scarcity of pain receptors in these areas produces a dull, poorly-localized pain of longer duration than cutaneous pain; examples include sprains and broken bones. Additional examples include the following.

### A. Excessive muscle tension

Excessive muscle tension can be caused, for example, by a sprain or a strain.

### B. Repetitive motion disorders

Repetitive motion disorders can result from overuse of the hands, wrists, elbows, shoulders, neck, back, hips, knees, feet, legs, or ankles.

### C. Muscle disorders

Muscle disorders causing somatic pain include, for example, a polymyositis, a dermatomyositis, a lupus, a fibromyalgia, a polymyalgia rheumatica, a macrophagic myofasciitis, and a rhabdomyolysis. Muscle pain can also be secondary to neurological and neuromuscular disorders including without limitation Parkinson's disease, Huntington's chorea, dystonias, tardive dyskinesias, drug-induced dyskinesias and dystonias, dyskinesias (paroxysmal), amyotrophic lateral sclerosis, multiple sclerosis, myoclonus, progressive supranuclear palsy, corticobasal degeneration, choreoathetosis, spasticity, Wilson disease, multiple system atrophy (including Shy-Drager syndrome, striatonigral degeneration and olivopontocerebellar atrophy), and hereditary spastic paraplegia (including familial spastic paraparesis, familial spastic paraplegia, hereditary spastic paraparesis, Strumpell-Lorraine syndrome, and Strumpell's disease).

### D. Myalgia

Myalgia is muscle pain and is a symptom of many diseases and disorders. The most common cause for myalgia is either overuse or overstretching of a muscle or group of muscles. Myalgia without a traumatic history is often due to viral infections. Longer-term myalgias may be indicative of a metabolic myopathy, some nutritional deficiencies or chronic fatigue syndrome.

### E. Infection

Infection can cause somatic pain. Examples of such infection include, for example, an abscess in the muscle, a trichinosis, an influenza, a Lyme disease, a malaria, a Rocky Mountain spotted fever, Avian influenza, the common cold, community-acquired pneumonia, meningitis, monkeypox, Severe Acute Respiratory Syndrome, toxic shock syndrome, trichinosis, typhoid fever, and upper respiratory tract infection.

### F. Drugs

Drugs can cause somatic pain. Such drugs include, for example, cocaine, statins for lowering cholesterol (such as atorvastatin, simvastatin, and lovastatin), and ACE inhibitors for lowering blood pressure (such as enalapril and captopril).

G. Prolonged nociceptive pain including without limitation to bone fracture pain, spinal stenosis, and post-surgical pain.

### V. Visceral pain

The compositions of the invention may be used to treat pain caused by or otherwise associated with any of the following visceral pain conditions. Visceral pain originates from body's viscera, or organs. Visceral nociceptors are located within body organs and internal cavities. The even greater scarcity of nociceptors in these areas produces pain that is usually more aching and of a longer duration than somatic pain. Visceral pain is extremely difficult to localise, and several injuries to visceral tissue exhibit "referred" pain, where the sensation is localised to an area completely unrelated to the site of injury.

Examples of visceral pain include the following.

### A. Functional visceral pain

Functional visceral pain includes, for example, chronic functional abdominal pain (CFAP), non-cardiac chest pain (NCCP), chronic abdominal pain, functional heartburn, functional dyspepsia, irritable bowel syndrome, painful bladder syndrome, vulvodynia, pelvic pain syndrome.

### B. Chronic gastrointestinal inflammation

Chronic gastrointestinal inflammation includes, for example, a gastritis, an inflammatory bowel disease, e.g., a Crohn's disease, an ulcerative colitis, a microscopic colitis, a diverticulitis and a gastroenteritis; an intestinal ischemia; a cholecystitis; an appendicitis; a gastroesophageal reflux; an ulcer, a nephrolithiasis, a pancreatitis and a hernia.

### C. Autoimmune pain

Autoimmune pain includes, for example, a sarcoidosis and a vasculitis.

### D. Organic visceral pain

Organic visceral pain includes, for example, pain resulting from a traumatic, inflammatory or degenerative lesion of the gut or produced by a tumor impinging on sensory innervation.

### E. Treatment-induced visceral pain

Treatment-induced visceral pain includes, for example, a pain attendant to chemotherapy therapy or a pain attendant to radiation therapy.

### VI. Referred pain

The compositions of the invention may be used to treat pain caused by or otherwise associated with any of the following referred pain conditions.

Referred pain arises from pain localized to an area separate from the site of pain stimulation. Often, referred pain arises when a nerve is compressed or damaged at or near its origin. In this circumstance, the sensation of pain will generally be felt in the territory that the nerve serves, even though the damage originates elsewhere. A common example occurs in intervertebral disc herniation, in which a nerve root arising from the spinal cord is compressed by adjacent disc material. Although pain may arise from the damaged disc itself, pain will also be felt in the region served by the compressed nerve (for example, the thigh, knee, or foot). Relieving the pressure on the nerve root may ameliorate the referred pain, provided that permanent nerve damage has not occurred. Myocardial ischaemia (the loss of blood flow to a part of the heart muscle tissue) is possibly the best known example of referred pain; the sensation can occur in the upper chest as a restricted feeling, or as an ache in the left shoulder, arm or even hand.

One can administer to a patient in need of treatment a composition comprising one or more serotonin-norepinephrine reuptake inhibitors and one or more alpha-2B receptor agonists. But one can also administer these compounds separately, administering one immediately after the other, or administering one within a short interval after the other (e.g., 5-15 minutes, or 15-30 minutes, or 30 minutes - 1 hour), or administering one within a longer interval after the other (e.g., 1-2 hours, 2-4 hours, 4-6 hours, 6-12 hours, or 1224 hours). One can also administer one compound more frequently than another, administering, for example, a serotonin-norepinephrine reuptake inhibitor one or more times daily and an alpha-2B receptor agonist two or more times daily (or vice versa).

### Examples

The invention is illustrated by the following example. This is provided for illustration only; many more embodiments are possible.

### Amitriptyline and two alpha-2B receptor agonists in the Chung model

Amitriptyline was administered intraperitoneally to rats at a minimally effective dose; that is, the compound was administered at a dose that, when administered alone, is the minimum dose required to relieve pain (this dose was calculated by finding, first, the dose that produced drug's maximum effect - that is, the dose that fully alleviated pain - and dividing that dose by three; the result is a dose that is slightly less than 50% of the dose that is required to produce the drug's maximum effect) The rats then immediately received ohe of two alpha-2B receptor agonists, compound No. 95 and compound no. No. 27, or received no additional compound, at all. Compound No. 95 and Compound No. 27 were administered intraperitoneally at ineffective doses; that is, these compounds were administered at doses that, when administered alone, are not effective in relieving pain. Pain reversal was then assessed using the Chung rat model of chronic pain. The model is described by Kim and Chung, Pain, 150, at 355-363 (1992) and in U.S. Patent No. 7,091,232, both of which are incorporated by reference herein.

The results of this experiment are shown in Table 2, below.

**Table 2 - Reuptake inhibitor alone versus reuptake inhibitor and alpha-2B receptor agonist**

| Example | Reuptake Inhibitor | Alpha-2B receptor agonist | Pain Reversal |
|---|---|---|---|
| 1 | Amitriptyline 30 µg/kg | None | 43% |
| 2 | Amitriptyline 30 µg/kg | Compound No. 95, 1 µg/kg | 80% |
| 3 | Amitriptyline 30 µg/kg | Compound No. 27, 10 µg/kg | 81% |

When administered alone, the minimally effective dose of amitriptyline reversed pain (allodynia) by 43%. When administered with what is otherwise an ineffective dose of Compound No. 95 and Compound No. 27 (respectively), the pain reversal nearly doubled, to 80% with Compound No. 95 and 81% with Compound No. 27.

## Claims

1. A pharmaceutical composition comprising a serotonin-norepinephrine reuptake inhibitor (SNRI) and an alpha-2B receptor agonist, wherein the serotonin-norepinephrine reuptake inhibitor is selected from the group consisting of amitriptyline, atomoxetine, desipramine, duloxetine, maprotiline, milnacipran, nefazodone, protriptyline, trimipramine, reboxetine, venlafaxine, and viloxazine and the alpha-2B receptor agonist is an alpha-2B/2C receptor agonist lacking significant activity at the alpha-2A receptor subtype, which comprises a compound having a structure selected from the group consisting of 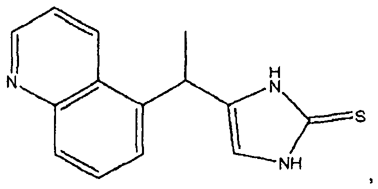 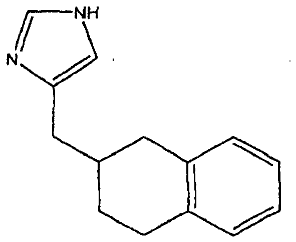 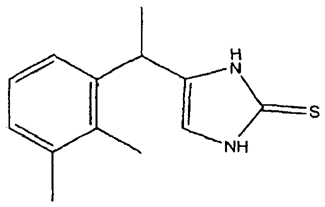 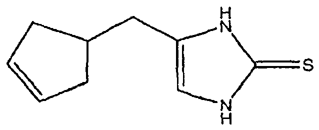 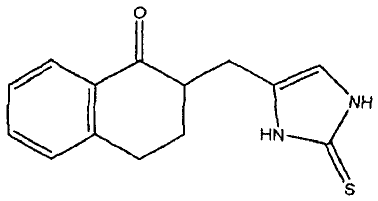 or a pharmaceutically acceptable salt of the compound.

2. A SNRI and an alpha-2B receptor agonist each as defined in claim 1 for use in the treatment of chronic pain.

3. The pharmaceutical composition of claim 1 or the compounds for use as defined in claim 2, wherein the serotonin-norepinephrine reuptake inhibitor or alpha-2B receptor agonist is synthetically produced.

4. The compounds for use according to claim 2 or 3, wherein the serotonin-norepinephrine reuptake inhibitor and the alpha-2B receptor agonist are administered as a single formulation.

5. The compounds for use according to claim 2 or 3, wherein a first formulation comprising the serotonin-norepinephrine reuptake inhibitor and a second formulation comprising the alpha-2B receptor agonist are administered at the same time.

6. The compounds for use according to claim 2 or 3, wherein a first formulation comprising the serotonin-norepinephrine reuptake inhibitor and a second formulation comprising the alpha-2B receptor agonist are administered at different times.

7. The compounds for use according to claims 2 or 3, wherein a first formulation comprising the serotonin-norepinephrine reuptake inhibitor is administered once daily and a second formulation comprising the alpha-2B receptor agonist is administered twice daily.

8. The compounds for use according to claim 2 or 3, wherein a first formulation comprising the serotonin-norepinephrine reuptake inhibitor is administered twice daily and a second formulation comprising the alpha-2B receptor agonist is administered once daily.

9. The compounds for use according to any one of claims 2-8, wherein at least one of the serotonin-norepinephrine reuptake inhibitor and the alpha-2B receptor agonist is administered at a dose that would be ineffective to relieve pain were the serotonin-norepinephrine reuptake inhibitor or alpha-2B receptor agonist administered alone.

## Patentansprüche

1. Pharmazeutische Zusammensetzung, umfassend einen Serotonin-Norepinephrin-Wiederaufnahmeinhibitor (SNRI) und einen alpha-2B-Rezeptor-Agonisten, wobei der Serotonin-Norepinephrin-Wiederaufnahmeinhibitor aus der Gruppe bestehend aus Amitriptylin, Atomoxetin, Desipramin, Duloxetin, Maprotilin, Milnacipran, Nefazodon, Protriptylin, Trimipramin, Reboxetin, Venlafaxin und Viloxazin ausgewählt ist und der alpha-2B-Rezeptor-Agonist ein alpha-2B/2C-Rezeptor-Agonist ist, dem die signifikante Aktivität am alpha-2A-Rezeptor-Untertyp fehlt und der eine Verbindung mit einer Struktur aufweist, die aus den Gruppen: 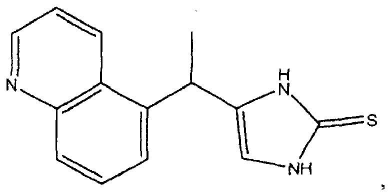 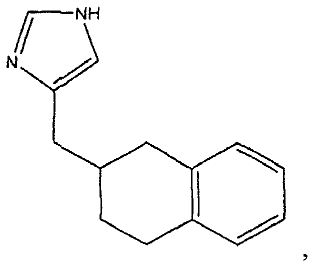 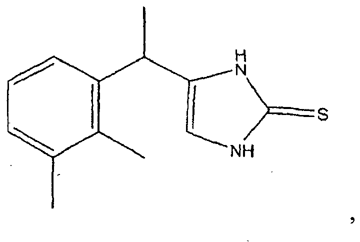 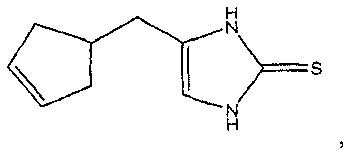 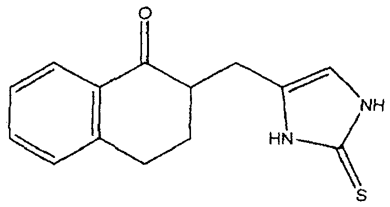 ausgewählt ist, oder ein pharmazeutisch annehmbares Salz der Verbindung.

2. SNRI und ein alpha-2B-Rezeptor-Agonist, jeweils wie in Anspruch 1 definiert, zur Verwendung bei der Behandlung von chronischen Schmerzen.

3. Pharmazeutische Zusammensetzung gemäss Anspruch 1 oder die Verbindungen zur Verwendung wie in Anspruch 2 definiert, wobei der Serotonin-Norepinephrin-Wiederaufnahmeinhibitor oder der alpha-2B-Rezeptor-Agonist synthetisch hergestellt sind.

4. Verbindungen zur Verwendung gemäss Anspruch 2 oder 3, wobei der Serotonin-Norepinephrin-Wiederaufnahmeinhibitor und der alpha-2B-Rezeptor-Agonist als einzelne Formulierung verabreicht werden.

5. Verbindungen zur Verwendung gemäss Anspruch 2 oder 3, wobei eine erste Formulierung, umfassend den Serotonin-Norepinephrin-Wiederaufnahmeinhibitor, und eine zweite Formulierung, umfassend den alpha-2B-Rezeptor-Agonisten, gleichzeitig verabreicht werden.

6. Verbindungen zur Verwendung gemäss Anspruch 2 oder 3, wobei eine erste Formulierung, umfassend den Serotonin-Norepinephrin-Wiederaufnahmeinhibitor, und eine zweite Formulierung, umfassend den alpha-2B-Rezeptor-Agonisten, zu unterschiedlichen Zeiten verabreicht werden.

7. Verbindungen zur Verwendung gemäss Anspruch 2 oder 3, wobei eine erste Formulierung, umfassend den Serotonin-Norepinephrin-Wiederaufnahmeinhibitor, einmal täglich verabreicht wird und eine zweite Formulierung, umfassend den alpha-2B-Rezeptor-Agonisten, zweimal täglich verabreicht wird.

8. Verbindungen zur Verwendung gemäss Anspruch 2 oder 3, wobei eine erste Formulierung, umfassend den Serotonin-Norepinephrin-Wiederaufnahmeinhibitor, zweimal täglich verabreicht wird und eine zweite Formulierung, umfassend den alpha-2B-Rezeptor-Agonisten, einmal täglich verabreicht wird.

9. Verbindungen zur Verwendung gemäss irgendeinem der Ansprüche 2 bis 8, wobei mindestens eines von Serotonin-Norepinephrin-Wiederaufnahmeinhibitor und alpha-2B-Rezeptor-Agonist in einer Dosis verabreicht wird, die zur Linderung von Schmerzen unwirksam wäre, wenn der Serotonin-Norepinephrin-Wiederaufnahmeinhibitor oder der alpha-2B-Rezeptor-Agonist alleine verabreicht würden.

## Revendications

1. Composition pharmaceutique comprenant un inhibiteur du recaptage de la sérotonine-norépinéphrine (SNRI) et un agoniste du récepteur alpha-2B, dans laquelle l'inhibiteur du recaptage de la sérotonine-norépinéphrine est choisi dans le groupe constitué de l'amitriptyline, de l'atomoxétine, de la désipramine, de la duloxétine, de la maprotiline, du milnaciprane, de la néfazodone, de la protriptyline, de la trimipramine, de la réboxétine, de la venlafaxine et de la viloxazine et l'agoniste du récepteur alpha-2B est un agoniste du récepteur alpha-2B/2C ne présentant pas d'activité significative au niveau du sous-type de récepteur alpha-2A, qui comprend un composé répondant à la structure choisie dans le groupe constitué de 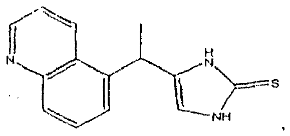 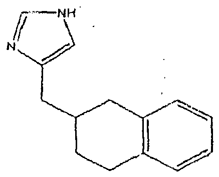 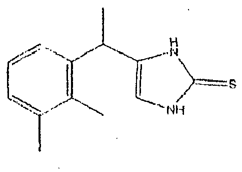 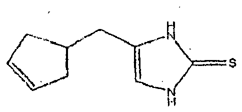 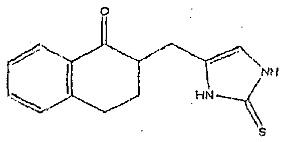 ou un sel pharmaceutiquement acceptable du composé.

2. SNRI et un agoniste du récepteur alpha-2B, chacun tel que défini dans la revendication 1 destinés à une utilisation dans le traitement de la douleur chronique.

3. Composition pharmaceutique selon la revendication 1 ou les composés destinés à une utilisation telle que définie dans la revendication 2, dans laquelle l'inhibiteur du recaptage de la sérotonine-norépinéphrine ou l'agoniste du récepteur alpha-2B est produit par voie synthétique.

4. Composés destinés à une utilisation selon la revendication 2 ou 3, dans lesquels l'inhibiteur du recaptage de la sérotonine-norépinéphrine et l'agoniste du récepteur alpha-2B sont administrés sous forme d'une seule formulation.

5. Composés destinés à une utilisation selon la revendication 2 ou 3, dans lesquels une première formulation comprenant l'inhibiteur du recaptage de la sérotonine-norépinéphrine et une seconde formulation comprenant l'agoniste du récepteur alpha-2B sont administrées en même temps.

6. Composés destinés à une utilisation selon la revendication 2 ou 3, dans lesquels une première formulation comprenant l'inhibiteur du recaptage de la sérotonine-norépinéphrine et une seconde formulation comprenant l'agoniste du récepteur alpha-2B sont administrées à des moments différents.

7. Composés destinés à une utilisation selon la revendication 2 ou 3, dans lesquels une première formulation comprenant l'inhibiteur du recaptage de la sérotonine-norépinéphrine est administrée une fois par jour et une seconde formulation comprenant l'agoniste du récepteur alpha-2B est administrée deux fois par jour.

8. Composés destinés à une utilisation selon la revendication 2 ou 3, dans lesquels une première formulation comprenant l'inhibiteur du recaptage de la sérotonine-norépinéphrine est administrée deux fois par jour et une seconde formulation comprenant l'agoniste du récepteur alpha-2B est administrée une fois par jour.

9. Composés destinés à une utilisation selon l'une quelconque des revendications 2 à 8, dans lesquels l'un au moins parmi l'inhibiteur du recaptage de la sérotonine-norépinéphrine et l'agoniste du récepteur alpha-2B est administré à une dose qui serait inefficace pour soulager la douleur si l'inhibiteur du recaptage de la sérotonine-norépinéphrine ou l'agoniste du récepteur alpha-2B était administré seul.
